# EUROPEAN PATENT APPLICATION

(11) **EP 1 840 572 A1**
(43) Date of publication of application: **03.10.2007**
(21) Application number: 06006775.8
(22) Date of filing: 30.03.2006
(51) Int. Cl.: G01N 33/53, G01N 33/80

(54) **A system for the enhancement of the detection of antibodies directed against red cell antigens**

(71) Applicant: STIFTUNG FÜR DIAGNOSTISCHE FORSCHUNG, CH-1785 Cressier sur Morat (CH)
(72) Inventor: Bashforth, David, 1586 Vallamand (CH)
(74) Representative: Müller-Boré & Partner Patentanwälte

(57) **Abstract**

The present invention relates to a method for detecting the presence or absence of one or more antibodies directed against one or more red cell antigens and a kit containing means for carrying out the above method.

## Description

The present invention relates to a method for detecting the presence or absence of one or more antibodies directed against one or more red cell antigens and a kit containing means for carrying out the above method.

The detection of antibodies directed against red cell antigens is of major importance in human blood transfusion practice. There are two primary applications: firstly, a screening procedure, i.e. antibody screening with subsequent identification in positive cases (panel), and secondly, the crossmatching of donor red cells against recipient plasma or serum prior to transfusion.

The most widely used technique is the indirect antiglobulin test (IAT) being a biphasic method, where in the first phase (i.e. sensitisation) red cells suspended in a phosphate based buffer are incubated with plasma or serum and eventually present antibodies bind with the corresponding antigen on the red cell surface. When the corresponding antigen is absent, no antibody is bound. In the second phase of the test, sensitised red cells are agglutinated by anti-human globulin (AHG), containing anti-IgG with or without antibodies against various fractions of complement. The sources of these antibodies are usually but not exclusively rabbit, goat or monoclonal.

In most current *in vitro* tests EDTA or citrate (in one of several forms) is used as anticoagulant agent, e.g. when testing erythrocytes in an EDTA containing buffer or when testing EDTA plasma. When testing humanized monoclonal antibodies directed against the red cell antigen Js^{b} in both IgM and IgG form, it was observed by direct agglutination and IAT, respectively, that these antibodies reacted when EDTA was absent but failed to react when it was present.

Thus, the problem underlying the present invention is to provide a new method for detecting the presence or absence of antibodies directed against red cell antigens.

The solution to the above technical problem is achieved by the embodiments characterized in the claims.

In particular, the present invention relates to a method for detecting the presence or absence of one or more antibodies directed against one or more red cell antigens, comprising the steps of
(a) incubating a first system containing one or more red cell antigens, and a second system containing one or more antibodies, in a buffer solution containing calcium ions in a non-complexed and/or non-chelated and/or non-precipitated form, a buffer salt and an anticoagulant, under antibody/antigen-complex forming conditions; and
(b) evaluating the antibody/antigen-complex formed in step (a) qualitatively and/or quantitatively.

The first system containing one or more red cell antigens, may be any *in vitro* system which contains at least one red cell antigen. In one embodiment of the present invention, the system may be a naturally occurring system, e.g. a solution selected from the group consisting of whole blood, serum, and plasma. Further, the system may comprise a solution containing red cell antigens derived from naturally occurring systems, e.g. a solution containing isolated blood compounds or processed blood products. In another embodiment of the present invention the system may comprise cells expressing one or more red cell antigens, e.g. a cell culture system. Methods for obtaining the above systems are known in the prior art.

The term "red cell antigen" as used herein relates to any red cell antigen including antigens that are located in the red cell membrane, e.g. antigens which protrude from the membrane of erythrocytes or antigens which are embedded in the membrane of erythrocytes. Further, the red cell antigen according to the present invention may be e.g. an antigen which is evenly distributed over the red cell surface as well as an antigen which is distributed in clusters over the red cell surface. Moreover, the red cell antigen according to the present invention may have any molecular structure including structures origininating carbohydrates, lipids, proteins, as well as combinations thereof.

The term "red cell antigen" as used herein also relates to any biologically active derivative of a red cell antigen having substantially the same biological function as the respective red cell antigen, e.g. a fragment or hapten of a red cell antigen which can be used for detecting the presence or absence of one or more antibodies directed against the whole red cell antigen.

In a preferred embodiment of the present invention, the red cell antigen is selected from the group consisting of those expressed within the ABO, Rh, Duffy, Kell, Kidd, Lutheran, P, MNS, I, LW, Gerbich, Diego, Dombrock, Cartwright, Cromer, Xg, Scianna, Chido-Rogers, Krops, Indian and other systems.

In an especially preferred embodiment of the present invention, the one or more red cell antigens are selected from the group consisting of Jka antigen, K antigen, Fya antigen, Fyb antigen, and S antigen.

The second system containing one or more antibodies, may be any *in vitro* system containing at least one antibody. In one embodiment of the present invention, the system may be a naturally occurring system such as a solution selected from the group consisting of whole blood, serum, and plasma. Further, the system may comprise a solution containing one or more antibodies derived from naturally occurring systems, e.g. a solution containing isolated blood compounds or processed blood products. In another embodiment of the present invention, the system may comprise cells expressing antibodies, e.g. a cell culture system. Methods for obtaining the above systems are known in the prior art.

The term "antibody" as used herein may be any antibody including biologically active fragments of an antibody having substantially the same biological function as the antibody, e.g. the ability to bind a specific antigen. In particular, the antibody according to the present invention can be selected from the group consisting of a naturally occurring antibody, a polyclonal antibody, a chimeric antibody, a monoclonal antibody, e.g. derived by conventional hybridoma techniques, and an antibody or antibody fragment obtained by recombinant techniques, e.g. phage display or ribosome display. In a preferred embodiment of the present invention, the antibody is a naturally occurring antibody.

In another preferred embodiment of the present invention, the one or more antibodies directed against one or more red cell antigens are alloantibodies.

The antibody of the present invention may belong to any immunoglobulin class. In a preferred embodiment of the present invention, the immunoglobulin class of the one or more antibodies directed against one or more red cell antigens is primarily IgG but may include IgM and IgA.

The buffer solution used in the method according to the present invention containing calcium ions in a non-complexed and/or non-chelated and/or non-precipitated form, may further contain any compound which does not negatively affect antibody/antigen-complex forming. In a preferred embodiment of the method according to the present invention the buffer solution is isotonic within the physiological limits of the pH value. It may be of normal or low ionic strength and may or may not contain nutrients for the long term storage of red cells.

As used herein the wording "buffer solution containing calcium ions in a non-complexed and/or non-chelated and/or non-precipitated form" means that the buffer solution contains free calcium ions which are not complexed, chelated or precipitated. Accordingly, the buffer solution used in the method according to the present invention does not contain an effective amount of an agent which complexes, chelates or precipitates calcium ions. In an especially preferred embodiment of the present invention, the buffer solution does not contain an effective amount of ethylenediamine tetraacetic acid (EDTA). If the buffer solution used in the method of the present invention contains an effective amount of an agent that complexes, chelates or precipitates calcium ions, calcium ions are present in the buffer solution in excess, which means that although part of the calcium ions are not freely available in the buffer solution, there still is an effective amount of calcium ions in the buffer solution which are still free, i.e. present as non-complexed, non-chelated and non-precipitated. Effectively, there is excess calcium which removes EDTA or citrate by ligand formation, preventing its inhibition of antibody/antigen interaction.

The calcium ions in the buffer solution used according to the method of the present invention are preferably present in the buffer solution as a calcium salt, e.g. a salt selected from the group consisting of calcium chloride and other highly soluble calcium salts which allow excess calcium ions to be present in solution.

According to the method of the present invention, the anticoagulation of red cells is achieved by using an anticoagulant which allows the forming of an antibody/antigen-complex in the presence of calcium ions in a non-complexed and/or non-chelated and/or non-precipitated form. In a preferred embodiment of the present invention the anticoagulant is not ethylenediamine tetraacetic acid (EDTA), citrate or any anticoagulant relying on the removal of calcium ions. In another preferred embodiment of the present invention the anticoagulant is heparin. In a more preferred embodiment of the method according to the present invention the anticoagulant is low molecular weight (LMW) heparin.

The buffer salts used in the buffer solution of the method according to the present invention may be any buffer salt as long as said buffer salt does not negatively effect the antibody/antigen-complex forming. In a preferred embodiment of the present invention the buffer salt is not a phosphate salt. In a preferred embodiment of the method according to the present invention, the buffer salt is selected from the group consisting of non phosphate buffers having a suitable pKa, e.g. TRIS, HEPES, Bis Tris Propane. In an especially preferred embodiment of the method according to the present invention, the buffer salt contained in the buffer solution comprises 3-(N-morpholino)propane sulfonic acid (MOPS).

In a preferred embodiment of the present invention, the buffer solution contains calcium ions in an amount ranging from about 1 to about 500 mM, more preferred about 2 to about 100 mM, more preferred about 5 to about 20 mM, and most preferred about 13.6 mM.

The incubation of the first system and the second system in the above-defined buffer solution is carried out under any conditions suitable for antibody/antigen-complex forming without any limitation. This comprises e.g. any suitable temperature, time period and agitation of the buffer solution. In a preferred embodiment of the present invention, the incubation is carried out at a temperature ranging from about 4°C to about 37°C for from about 5 to about 45 minutes. In a more preferred embodiment of the present invention, the incubation is carried out at about 37°C for about 15 minutes.

The antibody/antigen-complex formed can be evaluated qualitatively and/or quantitatively by methods well known in the art. Examples for the evaluation of the above mentioned complex are, but not limited to, for example the evaluation of blood agglutination, the use of a labelled antibody directed against one or more antibodies directed against one or more red cell antigens or one or more antibodies directed against one or more red cell antigens may be covalently linked to a detectable label which may be any suitable detectable label known in the art. Further, a labelled antibody directed against one or more red cell antigens may be used or one or more red cell antigens may be covalently linked to a detectable label which may also be any suitable detectable label known in the art. The detection method for measuring the detectable label can be, for example, selected from the group consisting of an enzyme assay, a chromogenic assay, a lumino assay, a fluorogenic assay, and a radioimmune assay. The reaction conditions to perform detection of the detectable label depend upon the detection method selected. It is within the knowledge of the person skilled in the art to choose the optimal parameters, such as buffer system, temperature and pH for the respective detection system to be used.

In a preferred embodiment of the present invention the method according to the present invention is an indirect antiglobulin test (IAT). The IAT according to the present invention may be carried out using any method known in the art for carrying out said test, including but not limited to slide, tube, and column technology, as well as microplate methods. In an especially preferred embodiment of the present invention the IAT is carried out comprising the following steps: (i) providing red cells, e.g. in a microtube; (ii) contacting said red cells with plasma or serum resulting in a respective mixture; (iii) incubating said mixture for about 8 to about 15 minutes at 37°C; (iv) centrifuging said mixture for about 4.5 to about 10 minutes; and scoring the reactions according to their grade of agglutination.

Preferred embodiments of the present invention the method according to the present invention are antibody screening and crossmatching. The crossmatching according to the present invention may be carried out using any method known in the art for carrying out said method. When carrying out crossmatching, the evaluation of the antibody/antigen-complex formed is the qualitatively and/or quantitatively evaluation of blood agglutination.

The present invention further relates to the use of the method according to the present invention for testing donor blood for a blood transfusion. Moreover, the present invention further relates to the use of the method according to the present invention for testing recipient blood for a blood transfusion.

The term "blood" as used herein includes any blood component and/or blood product suitable for carrying out antibody screening, such as e.g. serum, plasma, or other body fluids which may contain antibodies.

In one embodiment of the present invention one or more antibodies directed against one or more red cell antigens are immobilized on a solid substrate. In another preferred embodiment of the present invention one or more red cell antigens are immobilized on a solid substrate. The term "substrate" does not have any specific limitations, and relates, for example, to an insoluble polymer material, which can be an organic polymer, such as polyamide or a vinyl polymer (e.g. poly(meth)acrylate, polystyrene and polyvinyl alcohol, or derivatives thereof), a natural polymer such as cellulose, dextrane, agarose, chitin and polyamino acids, or an inorganic polymer, such as glass or metallohydroxide. The substrate can be in the form of a microcarrier, particles, membranes, strips, paper, film, pearls or plates, such as microtiter plates or microarrays. The term "microarray" as used herein means any arrangement of the antibodies or antigens in addressable locations on a substrate resulting in a so-called "biochip".

The antibodies or the antigens can be immobilized on the substrate directly by covalent coupling or via a carrier such as a linker molecule or an antibody immobilized on the substrate. In one specific example of the present invention, the antibody/antigen-complex formed may be detected using an Enzyme Linked Immunosorbent Assay (ELISA).

The antigens, antibodies, blood, blood components and blood products used in the present invention may be of any mammalian origin. In a preferred embodiment of the present invention, the antigens, antibodies, blood, blood components and/or blood products are derived from a mammal selected from the group consisting of e.g. rabbit, goat, mouse, sheep, pig, horse, chimpanzee, and human. In a more preferred embodiment of the present invention, the antigens, antibodies, blood, blood components and/or blood products are of human origin.

The present invention also relates to a kit containing means for carrying out the method according to the present invention. In particular, the kit may contain e.g. a buffer solution as defined above, a solid support as defined above, optionally having one or more antibodies or one or more antigens immobilized thereon, reaction containers, means for evaluating qualitatively and/or quantitatively an antibody/antigen-complex formed, including buffers, enzymes, enzyme substrates, when appropriate.

Additionally, the present invention relates to the a kit according to the present invention for diagnostic use. In a preferred embodiment of the present invention, the diagnostic use comprises the step of detecting the presence or absence of one or more antibodies directed against one or more red cell antigens in maternal blood to detect whether a pregnant mother has antibodies directed against react with fetal cells eventually causing hemolytic diseases of the newborn. In another preferred embodiment of the present invention the diagnosis comprises the step of detecting the presence or absence of one or more antibodies directed against one or more red cell antigens in the blood of a donor and/or a recipient of a blood transfusion to determine, whether a blood transfusion can be carried out, i.e. pre-transfusion testing. In another preferred embodiment of the present invention the diagnosis comprises the step of detecting the presence or absence of one or more antibodies directed against one or more red cell antigens in the of a donor and/or a recipient of an organ transplantation to determine, whether a transplantation will be successful, i.e. pre-transplantation testing.

The present invention provides a method which improves the detection of the presence of antibodies directed against red cell antigens thereby giving excellent results for clinically significant antibodies, e.g. alloantibodies. Accordingly, the present invention provides a highly reliable system for detecting the presence or absence of antibodies directed against red cell antigens having a significantly increased sensitivity and, thus, enhancing the reliability of the detection process of said antibodies. Accordingly, the present invention provides means for reducing the number of false negative results when testing clinical samples for the presence of antibodies directed against certain red cell antigens. Therefore the present invention advantageously increases the safety of e.g. blood typing, blood transfusions and transplantations.

The present invention will now be further illustrated in the following examples without being limited thereto.

### Examples

### Example 1: Model test systems

Model test systems were designed for both antibody screening and crossmatching.

### Antibody screening

In the standard system, antibody screening is performed using cells suspended at 0.8% NaCl in a low ionic strength phosphate preservative buffer containing sugars, purines, antibiotics, EDTA, sodium chloride and glycine, herein after designated as VP buffer which corresponds to the Ca buffer of Example 5.

In the test system, VP buffer is replaced by a preservative solution (designated as MOPS VP) containing calcium chloride (2 g/I), buffered by non-phosphate compounds such as MOPS to prevent the precipitation of calcium phosphate and an anticoagulant such as low molecular weight heparin which does not depend on the removal of calcium ions for its activity. The other ingredients are as for VP buffer with the exception of EDTA. The osmolarity, pH and conductivity are adjusted to be as for VP buffer (pH 6.7 to 7.0, osmolarity 300 mosm ± 5)

In these comparisons, standard production lots of screening cells were used suspended in VP and MOPS VP buffers. These two sets of three screening cells were labelled as A and B to avoid prejudice in the reading of reaction strength.

The above two sets of screening cells were tested against a series of plasmas and sera known to contain alloantibodies and neutral samples as negative controls by the following standard technique:
1. Pipette 50 µl of red cells into the relevant microtube.
2. Pipette 25 µl plasma or serum.
3. Incubate 15 minutes at 37°C.
4. Centrifuge in the DiaMed ID-Centrifuge for 10 minutes.
5. Reactions are scored according to their grade of coagulation.

### Example 2: Experiment 1

The antibody screening for clinically significant alloantibodies found in patient plasma is carried out as described in Example 1, above, using cells belonging to an in-house series 1 as starting material.

### Scoring system:

| Grade | Score |
|---|---|
| 4 | 4 |
| 3 | 3 |
| 2 | 2 |
| 1 | 1 |
| w | 0.5 |

**Table 1**

| Specificity | No. of antibodies | VP buffer | MOPS VP Buffer |
|---|---|---|---|
| Jka | 6 | 8 | 18.5 |
| K | 6 | 8.5 | 12 |
| Fya | 3 | 13 | 17 |
| Total | 15 | 29.5 | 47.5 |

### Example 3: Experiment 2

The antibody screening for clinically significant alloantibodies found in patient plasma is carried out as described in Example 1, above, in aggregate multiple testing, i.e. the sum of several runs, using cells belonging to an in-house series 2 as starting material using known weak antibodies.

### Scoring system:

| Grade | Score |
|---|---|
| 2 weak | 1.66 |
| 1 strong | 1.33 |
| 1 | 1 |
| 1 weak | 0.66 |
| weak | 0.5 |
| v. weak | 0.33 |
| trace | 0.2 |

**Table 2**

| Specificity | No. of antibodies | VP buffer | MOPS VP Buffer |
|---|---|---|---|
| Jka | 3 | 6.68 | 15.32 |
| K | 5 | 1.99 | 2.39 |
| Fya | 1 | 0.73 | 3.15 |
| Fyb | 1 | 0.4 | 4.72 |
| S | 1 | 4.48 | 8.32 |
| Total | | 14.28 | 33.9 |

### Example 4: Experiment 3

The antibody screening for clinically significant alloantibodies found in patient plasma is carried out as described in Example 1 above using cells belonging to external testing material as starting material.

### Scoring system:

| Grade | Score |
|---|---|
| 5 | 4 |
| 3 | 3 |
| 2 | 2 |
| 1 | 1 |
| w | 0.5 |

**Table 3**

| Specificity | No. of antibodies | VP buffer | MOPS VP Buffer |
|---|---|---|---|
| Jka | 2 | 1 | 4.5 |
| D | 3 | 1 | 7.5 |
| Fya | 5 | 2.5 | 9 |
| Jkb | 2 | 2 | 5.5 |
| Total | | 6.5 | 26.5 |

### Example 5: Antibody screening in patients

The antibody screening for clinically significant alloantibodies found in patient plasma is carried out as described in Example 1 above using patient's blood as starting material.

The Ca buffer used herein contains:
MOPS: 2.09 g/l
LMW heparin: 0.2 g/l
CaCl₂(2•H₂O): 2 g/l

### Patient NX****1Z

Anti-Jka: not detectable with standard technique, i.e. an incubation at 37°C for 15 minutes followed by a centrifugation of 10 minutes, weak positive using Ca buffer.

### Patient 35***29

Anti-D: not detectable with standard technique, mean grade 2 positive using Ca buffer.

### Patient NX****2Z

Anti-Fya: not detectable with standard technique, grade 1 positive using Ca buffer.

### Patient LC***5F

Anti-E: not detectable with standard technique, weak positive using Ca buffer.

### Patient LC***1E

Anti-S: not detectable with standard technique, weak positive using Ca buffer.

## Claims

1. A method for detecting the presence or absence of one or more antibodies directed against one or more red cell antigens, comprising the steps of
(a) incubating a first system containing one or more red cell antigens, and a second system containing one or more antibodies, in a buffer solution containing calcium ions in a non-complexed and/or non-chelated and/or non-precipitated form, a buffer salt and an anticoagulant, under antibody/antigen-complex forming conditions; and
(b) evaluating the antibody/antigen-complex formed in step (a) qualitatively and/or quantitatively.

2. The method according to any of the preceding claims, wherein the buffer solution contains about 1 to about 500 mM calcium ions in a non-complexed and/or non-chelated and/or non-precipitated form.

3. The method according to any of the preceding claims, wherein the buffer salt is not a phosphate-based buffer salt.

4. The method according to any of the preceding claims, wherein the anticoagulant is not ethylenediamine tetraacetic acid (EDTA), citrate or any anticoagulant relying on the removal of calcium ions.

5. The method according to any of the preceding claims being an indirect antiglobulin test (IAT).

6. The method according to any of claims 1 to 5 being a crossmatching or antibody screening procedure.

7. The method according to any preceding claims, wherein the one or more antibodies are immobilized on a solid substrate.

8. The method according to any of claims 1 to 7, wherein the one or more red cell antigens are immobilized on a solid substrate.

9. A kit containing means for carrying out the method as defined in any of the preceding claims.

10. The kit according to claim 9 for diagnostic use.
